(19) Europäisches Patentamt
European Patent Office
Office européen des brevets

(11) Numéro de publication: **0 083 886**
**B1**

(12) **FASCICULE DE BREVET EUROPEEN**

(45) Date de publication du fascicule du brevet: **22.05.85**

(21) Numéro de dépôt: **82402345.1**

(22) Date de dépôt: **21.12.82**

(51) Int. Cl.⁴: **C 07 C 121/75,** C 07 C 120/00, C 09 K 19/12 // C07C65/21, C07C69/84, C07C67/00

(54) **Composé organique du type benzoate de biphényle triplement substitué et son procédé de fabrication.**

(30) Priorité: **31.12.81 FR 8124563**

(43) Date de publication de la demande: **20.07.83 Bulletin 83/29**

(45) Mention de la délivrance du brevet: **22.05.85 Bulletin 85/21**

(84) Etats contractants désignés: **DE GB NL**

(56) Documents cités:
**EP - A - 0 011 002**
**FR - A - 2 179 180**
**FR - A - 2 297 201**
**US - A - 4 029 594**

(73) Titulaire: **THOMSON-CSF, 173, Boulevard Haussmann, F-75379 Paris Cedex 08 (FR)**

(72) Inventeur: **Dubois, Jean-Claude, THOMSON-CSF SCPI 173, bld Haussmann, F-75379 Paris Cedex 08 (FR)**
Inventeur: **Le Barny, Pierre, THOMSON-CSF SCPI 173, bld Haussmann, F-75379 Paris Cedex 08 (FR)**
Inventeur: **Billard, Jean, THOMSON-CSF SCPI 173, bld Haussmann, F-75379 Paris Cedex 08 (FR)**
Inventeur: **Le Berre, Serge, THOMSON-CSF SCPI 173, bld Haussmann, F-75379 Paris Cedex 08 (FR)**
Inventeur: **Thirant, Lydie, THOMSON-CSF SCPI 173, bld Haussmann, F-75379 Paris Cedex 08 (FR)**
Inventeur: **Beguin, Annie, THOMSON-CSF SCPI 173, bld Haussmann, F-75379 Paris Cedex 08 (FR)**

(74) Mandataire: **Wang, Pierre et al, THOMSON-CSF SCPI 173, Bld Haussmann, F-75379 Paris Cedex 08 (FR)**

**EP 0 083 886 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'emploi des cristaux liquides smectiques A dans les dispositifs de visualisation utilisant un effet mixte, thermique et électrique, est maintenant bien connu. Parmi les matériaux utilisables pour cet emploi, on trouve des dérivés du biphényle tels que le cyano-4-octyl-4'-biphényle qui présentent une phase smectique A et une phase nématique. D'autres brevets pris par la titulaire concernent des matériaux smectiques A présentant ou non une phase nématique lors des transitions de phases smectique-isotrope. La présente invention concerne des dopants destinés à être incorporés à ces matériaux. Ces dopants entraînent en particulier une augmentation du contraste, une diminution de la puissance de commande et de la tension vidéo.

Plus particulièrement, la présente invention concerne des dopants utilisables avec des cristaux liquides smectiques dans les dispositifs de visualisation thermo-optiques avec ou sans assistance d'un champ électrique de commande. Le matériau smectique obtenu, après adjonction d'un ou de plusieurs dopants selon l'invention, peut présenter ou non une phase nématique.

Ces dopants formés de composés organiques sont caractérisés en ce qu'ils répondent à la formule générale suivante:

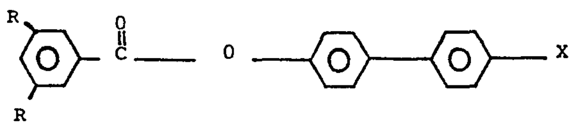

où R représente un groupement organique du type alkyle, alcoxy ou alkylcarboxylate(ester) comportant 1 à 15 atomes de carbone, et où X désigne un radical nitrile ou halogène.

Ces corps sont désignés par exemple par des expressions du genre: dialkyl ou dialkoxy-3,5 de p-cyano- ou p-halogénobenzoate de biphényle.

On donne ci-après un procédé général de fabrication de ces corps, puis on décrit le mode opératoire de quelques étapes caractéristiques pour un cas particulier. Suivra un exemple d'application à la visualisation par des systèmes à cristaux liquides smectiques.

*Procédé général de fabrication*

Les dopants peuvent être élaborés à partir d'un produit de base tel que l'acide 3,5-dihydrobenzoïque de formule:

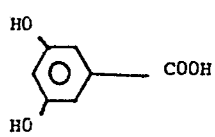

1. estérification de l'acide 3,5-dihydroxybenzoïque afin d'obtenir du dihydroxy-3,5-benzoate de méthyle de formule:

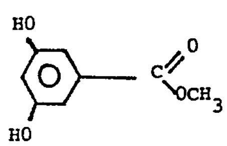

2. alkylation du précédent produit pour fixer le radical R et obtenir un produit ayant la formule générale suivante:

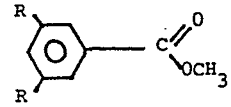

3. acidification du produit précédent pour obtenir le composé suivant:

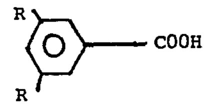

4. chloruration de l'acide précédent élaboré pour obtenir le chlorure d'acide:

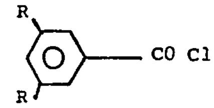

5. estérification à partir du chlorure d'acide pour obtenir le produit final:

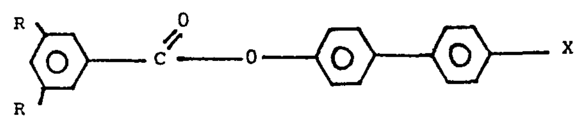

A titre d'exemple, on va donner le mode opératoire d'un dopant selon l'invention: le di-octyloxy-3,5-benzoate de 4',4''-cyanophénylphényle

*Mode opératoire*

*1. Synthèse du dihydroxy-3,5-benzoate de méthyle*

Dans un erlenmeyer de 250 ml, muni d'un agitateur magnétique, on dissout 5,9 g d'acide 3,5-dihydroxybenzoïque dans 50 ml d'éther éthylique. On ajoute ensuite une solution éthérée de diazométhane préparée selon une méthode connue («Vogel's Text Book of Pratical Organic Chemistry», 4e éd., Longman, Londres et New York, p. 291) jusqu'à l'obtention de la coloration jaune caractéristique d'un excès de diazométhane. Un précipité apparaît, qui est séparé du milieu réactionnel par filtration. Le filtrat est évaporé à sec sous vide. Le produit brut est dissous dans un mélange chloroforme 90/méthanol 10, puis précipité par l'éther de pétrole. On filtre et on sèche sous vide. On obtienr 5,17 g d'ester I. Le rendement de cette réaction est de 80%.

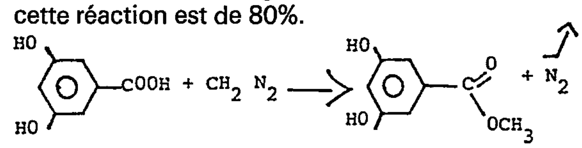

*2. Synthèse du dioctyloxy-3,5-benzoate de méthyle*

Dans un erlenmeyer de 500 ml, on introduit 200 ml d'alcool éthylique, puis 2,3 g de sodium décapé. Un réfrigérant à reflux est adapté à l'erlenmeyer et le milieu réactionnel est agité au moyen d'un barreau aimanté. Lorsque l'éthylate de sodium est formé, on ajoute 8,4 g de dihydroxy-3,5-benzoate de méthyle en agitant. Dès que le milieu est devenu homogène, on additionne goutte à goutte 20,26 g de bromure d'octyle distillé. On chauffe à reflux pendant 20 h.

Le milieu réactionnel est transvasé dans un ballon de 500 ml et l'alcool éthylique est distillé. Puis on ajoute 100 ml d'eau permutée et on distille jusqu'à obtention de la température de 100° C dans les vapeurs. L'ester II est extrait à l'éther, puis purifié par chromatographie liquide (éluant hexane 50/toluène 50), le point de fusion étant de 36,5° C.

Le rendement de cette réaction est de 30%.

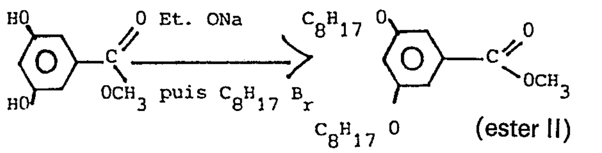

(ester II)

*3. Synthèse de l'acide dioctyloxy-3,5-benzoïque*

Dans un ballon de 1 l, on dissout à chaud 39 g de potasse dans 400 ml d'alcool éthylique, puis on ajoute 12,5 g d'ester II et on chauffe à reflux pendant 2 h. Le milieu réactionnel est concentré sous vide. On y ajoute de l'eau et on acidifie la phase aqueuse ainsi obtenue. L'acide III est extrait à l'éther éthylique, puis recristallisé dans l'alcool à −18° C. Le rendement de la méthode est de 90%.

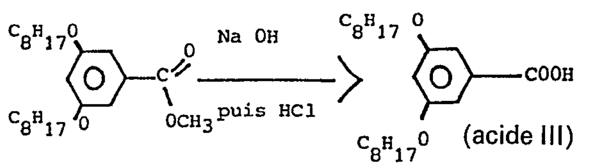

(acide III)

*4. Synthèse du chlorure de l'acide dioctyloxy-3,5-benzoïque*

L'acide III est transformé en chlorure d'acide IV par traitement à reflux du chlorure de thionyle.

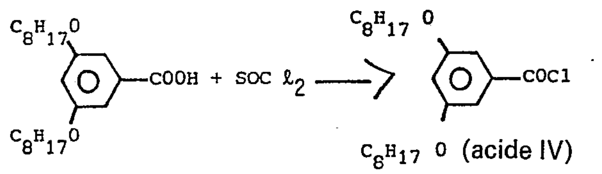

(acide IV)

*5. Synthèse du dioctyloxy-3,5-benzoate de 4',4''-cyanophénylphényle*

Dans un ballon de 100 ml contenant 11,5 g de chlorure d'acide IV, on introduit une solution de 5,67 g de 4-hydroxy-4'-cyanobiphényle dans 40 ml de pyridine. On agite pendant 48 h à température ambiante. Le mélange réactionnel est coulé sur 200 g de glace. On acidifie à pH=3, puis on extrait l'ester V à l'éther. La phase éthérée est lavée à l'eau puis séchée. L'ester est purifié par chromatographie liquide sous pression (éluant toluène), puis par recristallisation dans l'éthanol (F = 86,5° C). Le rendement de cette méthode est de 60%.

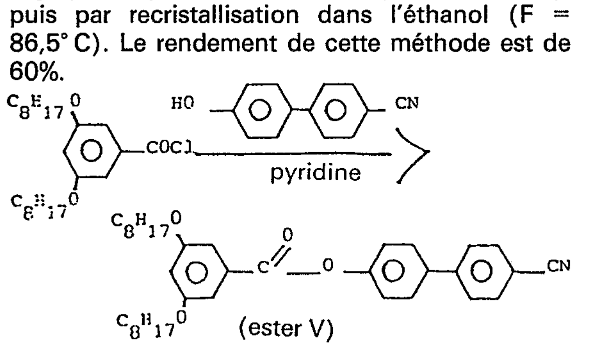

(ester V)

Lorsque l'on introduit un dopant répondant à la formule générale citée plus haut dans des cristaux liquides smectiques, on constate une modification des qualités optiques de ces cristaux. Ces dopants entraînent, dans les dispositifs de visualisation, une augmentation du contraste, une diminution de la puissance de commande et une diminution de la tension vidéo. Ils sont utilisables dans des dispositifs thermo-optiques avec ou sans assistance du champ électrique. Il est avantageux de les employer dans des dispositifs utilisant l'effet mixte thermique et électrique. L'introduction d'un ou de plusieurs dopants dans un cristal liquide smectique provoque, à partir d'un certain pourcentage, la suppression de la phase nématique, mais le nouveau matériau ainsi obtenu peut toujours être commandé par un champ électrique lors du passage de la phase isotrope à la phase smectique, et cela pour des valeurs de tensions de commande inférieures à celles mises en œuvre pour le même matériau smectique de base sans dopants.

A titre d'exemple non limitatif, on peut introduire le dopant dont l'élaboration a été décrite précédemment avec le cyano-4-octyl-4'-biphényle qui est un cristal liquide smectique fréquemment utilisé dans les dispositifs de visualisation. Les transitions de phase de ce cristal liquide sont: K 21,5 $S_A$ 33,5 N 40,5 I (les températures étant exprimées en degrés Celsius).

Lorsque l'on introduit dans ce cristal liquide le dopant considéré (dioctyloxy-3,5-benzoate de 4',4''-cyanophénylphényle), on modifie les transitions de phase selon la diagramme de phase de la figure donnée en annexe. Les ordonnées de ce diagramme sont graduées en température (de −10 à +90° C). L'abscisse est graduée de 0 à 100% de dopant en poids dans le mélange cristal liquide/dopant. Pour l'ordonnée A, le cristal contient 0% de dopant; pour l'ordonnée B, il contient 100% de dopant.

Sur ce diagramme, on retrouve les transitions de phase du matériau smectique de base (cyano-4-octyl-4'-biphényl) sans dopant: ce sont les points C (21,5° C), D (33,5° C) et E (40,5° C). On constate que plus le pourcentage de dopant dans le mélange cristal liquide/dopant augmente, plus l'amplitude de la phase nématique décroît pour s'annuler vers environ 5% de dopant (droite zz' élevée à partir de l'axe des abscisses).

Le contraste, dans ce genre de dispositif de visualisation, croît proportionnellement à la quantité de dopant introduit. L'augmentation du contraste en fonction du pourcentage de dopant pourrait s'expliquer par une analogie avec le phénomène de cristallisation à partir de germes. L'introduction de dopants appropriés à l'intérieur d'un cristal liquide smectique amènerait une multiplication des défauts à l'intérieur du matériau, ce qui aurait pour conséquence de diminuer la taille des zones diffusantes et donc d'augmenter le contraste. Pratiquement, les proportions de dopants ajoutées varient de 1% à des proportions plus importantes, de l'ordre de 15%, lors de l'utilisation de ce produit dopé dans des dispositifs de visualisation à accès matriciel. Le contraste obtenu est multiplié par un facteur 2 ou 3 (pour les plus fortes concentrtions de dopant) et la tension vidéo est diminuée dans le même rapport. La puissance de commande nécessaire pour échauffer le cristal liquide pour le faire passer de la phase smectique à la phase isotrope est diminuée du fait de la réduction de l'amplitude thermique de la phase nématique ou même de sa suppression.

La forme de la molécule du composé organique jouant le rôle de dopant a une action déterminante au sein du cristal liquide smectique où elle est introduite, en particulier à cause de la nature des groupements organiques R.

On obtient des résultats similaires avec un mélange de cristaux liquides donné également à titre d'exemple:

$C_8H_{17}$ 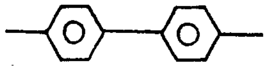 CN: 60% en moles

$C_{10}H_{21}$ 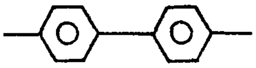 CN: 40% en moles

Ce mélange présente les transitions de phase suivantes: K9 $S_A$ 42,9 N 44,8 I. On y incorpore un dopant selon l'invention (le dioctyloxy-3,5-benzoate de 4',4''-cyanophénylphényle) afin de réduire l'amplitude comme l'indique le tableau ci-dessous où les températures ont été relevées pendant le chauffage du mélange dopé, de la phase smectique à la phase isotrope.

| Dopant (% en poids) | $S_A$ | N | I |
|---|---|---|---|
| 0 | . 42,9 | . 44,8 | . |
| 2 | . 42,4 | 43,6 | . |
| 5 | . 41,4 | . 42 | . |
| 7,5 | . | 40,5 | . |
| 10 | . | 40,2 | . |

Sans dopant, le mélange présente une phase nématique qui possède une amplitude thermique de 1,9° C. L'adjonction du dopant a eu pour effet de réduire cette amplitude: 1,2° C pour 2% de dopant en poids du mélange dopé, 0,6° C pour 5% de dopant. A partir de 7,5% de dopant, la phase nématique est supprimée. Comme précédemment, le contraste obtenu dans les dispositifs thermo-optiques croît également en fonction de la proportion de dopant.

Il entre aussi dans le cadre de l'invention d'utiliser le composé selon l'invention dans des dispositifs de visualisation à cristaux liquides smectiques utilisant uniquement l'effet thermique pour commander l'orientation des molécules, par exemple à l'aide d'un faisceau laser modulé en intensité.

Le composé organique selon l'invention permet de doper des cristaux liquides smectiques afin de réduire l'amplitude thermique de la phase nématique et même de la supprimer, tout en conservant la possibilité d'utiliser ces matériaux smectiques dans des dispositifs de visualisation à effet mixte thermique et électrique. L'introduction de ce dopant procure le triple avantage d'augmenter le contraste, de diminuer la puissance de commande et de diminuer la tension vidéo.

## Revendications

1. Composé organique du type benzoate de biphényle, caractérisé en ce qu'il répond à la formule chimique suivante:

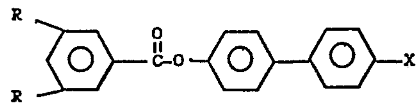

dans laquelle R représente un groupement alkyle, alcoxy ou alkylcarboxylate de formule $C_nH_{2n+1}$, $C_nH_{2n+1}-O-$ ou

$$C_nH_{2n+1}-\overset{O}{\overset{\|}{C}}-O-,$$

où n est un entier de 1 à 15, X désignant un radical nitrile ou halogène.

2. Composé organique selon la revendication 1, caractérisé en ce que les symboles sont les suivants: R = $C_8H_{17}O-$; X = CN.

3. Utilisation d'un composé organique selon l'une des revendications 1 ou 2, comme dopant d'un cristal liquide smectique.

4. Utilisation d'un composé organique selon l'une des revendications 1 ou 2, en proportions suffisantes, comme dopant d'un cristal liquide smectique afin de supprimer la phase nématique lors de la transition de phase smectique-isotrope.

5. Dispositif de visualisation contenant un composé organique selon l'une des revendications 1 ou 2, comme dopant d'un cristal liquide smectique.

6. Procédé de fabrication d'un composé organique selon la revendication 1, caractérisé en ce qu'il comporte:

a) une première étape de synthèse du dihydroxy-3,5-benzoate de méthyle à partir de l'acide 3,5-dihydroxybenzoïque et du diazométhane,

b) alkylation du produit de l'étape a en présence d'éthylate de sodium pour obtenir l'ester suivant:

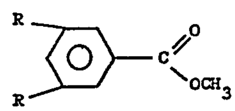

c) à partir du produit obtenu à l'étape b, on obtient un acide répondant à la formule suivante

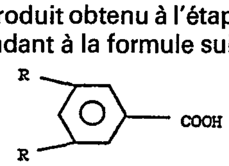

par action successive de potasse et d'acide chlorhydrique,

d) chloruration de l'acide obtenu à l'étape c par traitement à reflux par le chlorure de thionyle $SOCl_2$,

e) action sur le chlorure d'acide obtenu à l'étape d d'un biphényle de formule

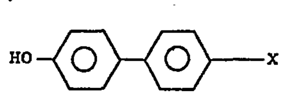

en présence de pyridine, suivie d'une purification par chromatographie et recristallisation de l'ester résultant.

## Patentansprüche

1. Organische Verbindung des Typs Benzoesäurebiphenylylester, gekennzeichnet durch die folgende chemische Formel:

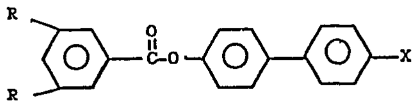

worin R eine Alkyl-, Alkoxy- oder Alkylcarboxylatgruppe der Formel $C_nH_{2n+1}$, $C_nH_{2n+1}-O-$ oder

$$C_nH_{2n+1}-\overset{\overset{O}{\|}}{C}-O-,$$

worin n eine ganze Zahl von 1 bis 15 ist, bedeutet und X einen Nitrilrest oder Halogen bezeichnet.

2. Organische Verbindung nach Anspruch 1, dadurch gekennzeichnet, dass die Symbole die folgende Bedeutung haben: $R = C_8H_{17}O-$; X = CN.

3. Verwendung einer organischen Verbindung nach einem der Ansprüche 1 oder 2 als Dotierungsmittel eines smektischen flüssigen Kristalls.

4. Verwendung einer organischen Verbindung nach einem der Ansprüche 1 oder 2 in genügenden Anteilen als Dotierungsmittel eines smektischen flüssigen Kristalls zur Unterdrückung der nematischen Phase beim smektischen-isotropen Phasenübergang.

5. Displayvorrichtung, welche eine organische Verbindung nach einem der Ansprüche 1 oder 2 als Dotierungsmittel eines smektischen flüssigen Kristalls enthält.

6. Verfahren zur Herstellung einer organischen Verbindung nach Anspruch 1, gekennzeichnet durch die folgenden Verfahrensstufen:

a) eine erste Stufe der Synthese des Methyl-3,5-dihydroxybenzoats aus 3,5-Dihydroxybenzoesäure und Diazomethan,

b) Alkylierung des Produkts der Stufe (a) in Gegenwart von Natriumethylat, um den folgenden Ester zu erhalten:

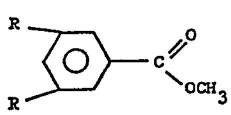

c) ausgehend von dem in Stufe (b) erhaltenen Produkt erhält man eine Säure der allgemeinen Formel:

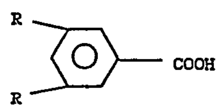

durch aufeinanderfolgende Einwirkung von Pottasche und Chlorwasserstoffsäure,

d) Chlorierung der in Stufe (c) erhaltenen Säure durch Behandlung unter Rückfluss mit Thionylchlorid $SOCl_2$,

e) Einwirkung eines Biphenyls der Formel:

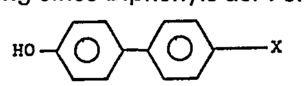

in Gegenwart von Pyridin auf das in Stufe (d) erhaltenen Säurechlorid und anschliessende chromatographische Reinigung und Umkristallisation des erhaltenen Esters.

## Claims

1. An organic compound of the biphenylylbenzoate type, characterized by the following chemical formula:

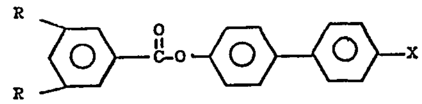

in which R represents an alkyl, alkoxy or alkyl carboxylate group of formula $C_nH_{2n+1}$, $C_nH_{2n+1}-O-$ or

$$C_nH_{2n+1}-\overset{\overset{O}{\|}}{C}-O-,$$

in which n is an integer ranging from 1 to 15 and X designates a nitrile radical or halogen.

2. An organic compound according to Claim 1, for which the symbols are as follows: $R = C_8H_{17}O-$; X = CN.

3. Use of an organic compound according to one of Claims 1 or 2 as a dopant for a smectic liquid crystal.

4. Use of an organic compound according to one of Claims 1 or 2 in adequate proportions as a dopant for a smectic liquid crystal in order to eliminate the nematic phase during the smectic-isotropic phase transition.

5. A display means containing an organic compound according to one of Claims 1 or 2 as a dopant of a smectic liquid crystal.

6. A process for the preparation of an organic compound according to Claim 1, characterized by:

(*a*) a first stage of synthesis of the methyl-3,5-dihydroxybenzoate from the 3,5-dihydroxybenzoic acid and diazomethane,

(*b*) alkylation of the product of stage (*a*) in the presence of sodium ethylate to obtain the following ester:

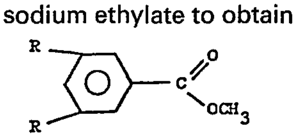

(*c*) from the product obtained in stage (*b*) is obtained an acid in accordance with the following formula:

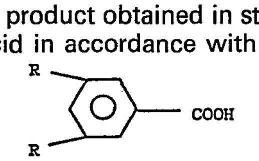

by the successive action of potash and hydrochloric acid,

(*d*) chlorination of the acid obtained in stage (*c*) by refluxing with thionyl chloride $SOCl_2$,

(*e*) action on the acid chloride obtained in stage (*d*) of a biphenyl of the formula:

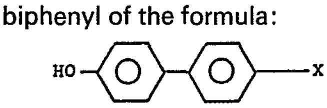

in the presence of pyridine, followed by chromatographic purification and recrystallization of the resulting ester.

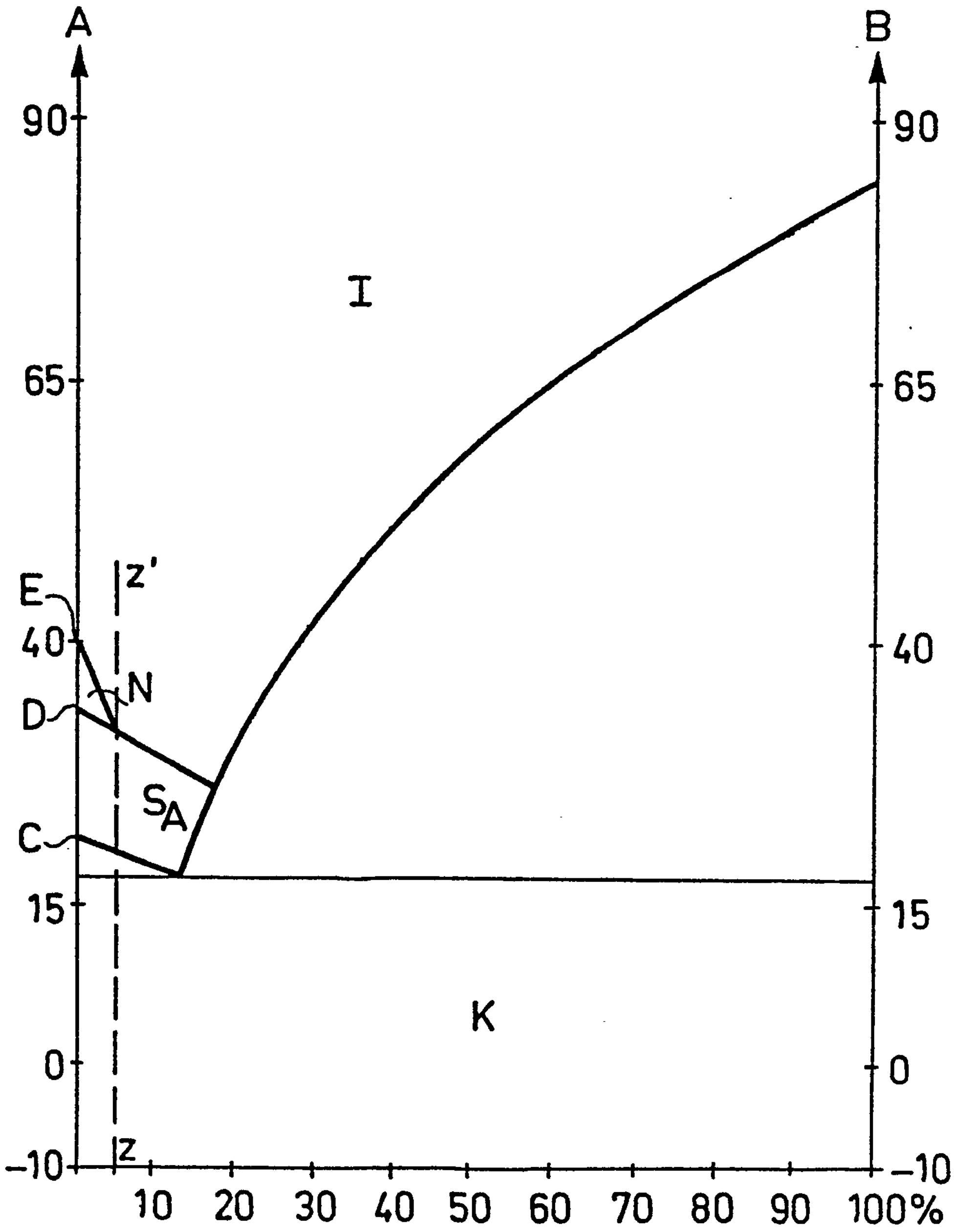
A
B
90
65
40
15
0
-10
I
K
E
D
C
N
z'
z
$S_A$
10
20
30
40
50
60
70
80
90
100%